# EUROPEAN PATENT SPECIFICATION

(11) **EP 4 472 540 B1**
(45) Date of publication and mention of the grant of the patent: **01.04.2026**
(21) Application number: 22705295.8
(22) Date of filing: 03.02.2022
(51) Int. Cl.: A61B 18/26, A61B 18/24

(54) **URETEROSCOPE DEVICES AND SYSTEMS HAVING A FIBER TIP POSITION SENSOR**
URETHROSKOPVORRICHTUNGEN UND SYSTEME MIT FASERSPITZENPOSITIONSSENSOR
DISPOSITIFS ET SYSTÈMES D'URÉTÉROSCOPE AYANT UN CAPTEUR DE POSITION DE POINTE DE FIBRE

(43) Date of publication of application: 11.12.2024
(73) Proprietor: C.R. BARD, INC., Murray Hill New Jersey 07974 (US)
(72) Inventor: CHO, Young, Covington, Georgia 30016 (US); HESS, Paul Robert, Snellville, Georgia 30078 (US); O BRIEN, Killian, Dublin, D08 E0A9 (IE)
(74) Representative: M. Zardi & Co S.A.
(86) International application number: PCT/US2022/015078
(87) International publication number: WO 2023/149885

(56) References cited:
- WO-A1-2021/026136
- US-A1- 2021 038 309
- US-A1- 2021 038 310
- US-A1- 2021 236 187

## Description

### BACKGROUND

Endoscopes of small size are desired in many industrial and medical applications. For example, when natural orifices and lumens of a human body are small, small endoscopes are used for insertion through such orifices and lumens to target locations within the body. For single incision laparoscopy, smaller endoscopes are preferred to provide an inside-the-body view of the surgical site, particularly when the incision itself is of minimal dimensions. Sometimes, patients may feel irritation when an endoscope is being inserted into his or her body, and a smaller endoscope may mitigate such unpleasant experience and may minimize trauma to the patient. Moreover, a physician may improve diagnostic and procedural protocols with a smaller endoscope. For example, transnasal endoscopy may sometimes replace trans-oral endoscopy. Endoscopes such as those disclosed in US 2021/038309 A1 may be used with laser fibers in procedures known as laser lithotripsy.

### SUMMARY

The invention is defined in the appended set of claims. Methods mentioned hereinafter do not form part of the present invention.

Embodiments disclosed herein are related to ureteroscope systems. In an embodiment, a ureteroscope system is disclosed. The ureteroscope system includes a handpiece having a distal end region, a proximal end region, an interior region, a working channel port, a first portion of a working channel in fluid communication with the working channel port, and a cable port. The ureteroscope system includes a catheter extending from the distal end region of the handpiece, the catheter including a second portion of the working channel in fluid communication with the working channel port and the first portion of the working channel, a distal end defining a working channel opening in fluid communication with the working channel. The ureteroscope system includes one or more sensors positioned and configured to detect a position of at least a portion of a laser fiber. The ureteroscope system includes a processor in communication with the one or more sensors and configured to determine if an emitting end of the laser fiber configured to emit laser light is outside the working channel and spaced at least a predetermined distance from the distal end of the catheter such that laser light emitting from the emitting of the laser fiber does not damage the catheter and/or the handpiece.

In an embodiment, a method of using a laser fiber with an ureteroscope is disclosed. The method includes inserting a catheter of the ureteroscope into a urethra of a subject to a selected position. The method includes feeding or inserting the laser fiber into a working channel port on a handpiece of the ureteroscope and through a working channel positioned at least partially in the handpiece and the catheter and in fluid communication with the working channel port. The method includes determining if an emitting end on the laser fiber is outside the working channel and spaced at least a predetermined distance from a working channel opening in a distal end of the catheter distal to the handpiece using one or more sensors and a processor in communication with the one or more sensors. The method includes emitting a laser light from the emitting end of the laser fiber if the emitting end on the laser fiber is determined to be outside the working channel and spaced at least the predetermined distance from the working channel.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** illustrates a digital ureteroscope as part of an endoscope system, according to an embodiment.
**FIG. 1B** illustrates an end view of a distal end of the catheter of the ureteroscope of **FIG. 1A****,** according to an embodiment.
**FIG. 2** is a side view of the handpiece of the ureteroscope of **FIG. 1A** with a side of the handpiece removed, according to an embodiment.
**FIG. 3** is a side view of the handpiece of the ureteroscope of **FIG. 1A****,** according to an embodiment.
**FIG. 4** is a flow diagram of a method of operating a ureteroscope system, according to an embodiment.
**FIG. 5** is a schematic of a controller that may be used in systems and methods described herein, according to an embodiment.

### DETAILED DESCRIPTION

Embodiments disclosed herein relate to devices, systems, and methods of using an endoscope, such as a ureteroscope. Ureteroscopes are sometimes used with a laser fiber during, for example, a surgical procedure known as a laser lithotripsy to remove stones from the urinary tract of a subject. For example, a ureteroscope may be used with a laser fiber to remove stones from the kidney, ureter, bladder, and/or urethra of a subject. During a laser lithotripsy procedure, a catheter of the ureteroscope is inserted into the urinary tract of the subject to locate the stone. The laser fiber *(e.g.,* an optical fiber) is inserted through the working channel of the ureteroscope, and laser light is emitted from the laser fiber to fragment or break apart the stone. The stone is fragmented by the laser light, and the remaining pieces may be collected in a basket and/or washed out of the urinary tract.

During use of the laser fiber with the ureteroscope, the laser light can damage the ureteroscope if the laser light is emitted while the emitting end of the laser fiber that emits the laser light is still in the working channel of the scope. At least one, some, or all of the embodiments of ureteroscope systems described herein may result in the technical effect of preventing or inhibiting the laser light from emitting (e.g., firing) while the emitting end of the laser fiber is still in the working channel of the ureteroscope and/or too close to the working channel. Thus, embodiments of the ureteroscope systems described herein may result in the in the technical effect of preventing damage to the ureteroscope system and/or the laser fiber by ensuring the laser light is emitted from the emitting end of the laser fiber only when the emitting end is outside the working channel and spaced a predetermined distance from the distal end of the catheter. In some embodiments, a processor uses data collected from an image sensor at or near the distal end of the catheter to determine if the emitting end of the laser fiber is located outside of working channel and spaced a predetermined distance from the distal end of the catheter. For example, the processor may determine or otherwise measure a color of the laser fiber *(e.g.,* blue) on a display that is displaying images or video from the image sensor. When the processor determines enough of the color of the laser fiber is visible on the screen to indicate that the emitting end of the laser fiber is outside the working channel and spaced the predetermined distance from distal end of the catheter, an alert may be displayed or otherwise signaled, thereby providing the technical effect of an indication that it is safe to fire or emit the laser light from the emitting end of the laser fiber.

In some embodiments, the ureteroscope may include a sensor positioned inside or proximate to the handpiece of the ureteroscope to detect the laser fiber passing through the working channel and/or the working channel port. The laser fiber and/or a jacket on the laser fiber may include one or more markers (e.g., dots, lines, or barcodes). The sensor may be positioned to detect the one or more markers and a processor associated with the sensor may interpret and/or count the markers passing through the working channel, then determine when the emitting end of the laser fiber is outside the working channel and spaced the predetermined distance from the distal end of the catheter.

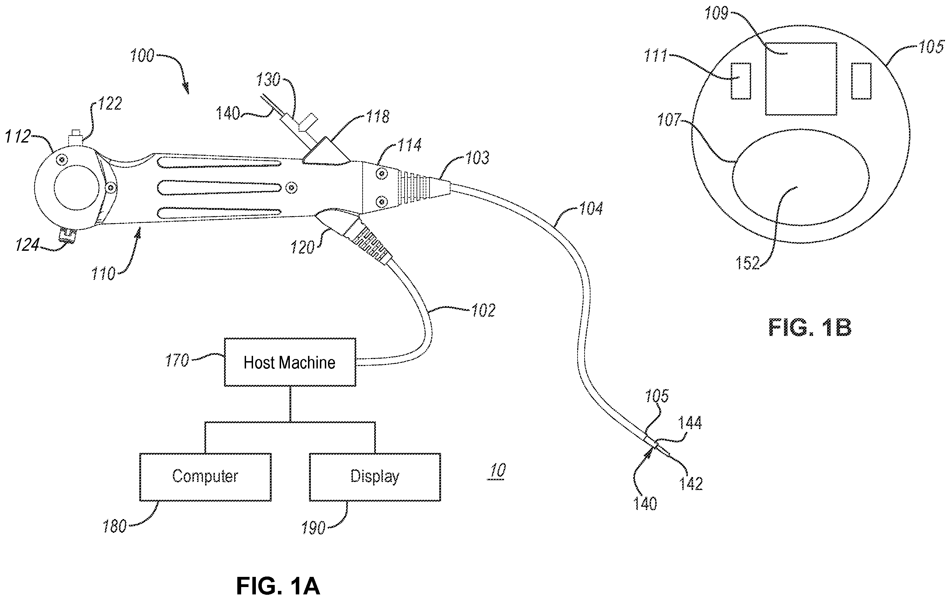

**FIG. 1A** illustrates an endoscopic system 10. In an embodiment, the endoscopic system 10 includes a digital ureteroscope 100 operably coupled to one or more electronic devices, such as a host machine 170 and one or more external terminals including a display 190 and a computer 180. The ureteroscope 100 may be operably coupled to one or more of the host machine 170, the computer 180, or the display 190 wirelessly or through a cable 102. The ureteroscope 100 includes a catheter 104 and a handpiece 110 having a catheter end 114 *(e.g.,* distal end region) and a control end 112 *(e.g.,* proximal end region) opposite to the catheter end 114. As the control end 112 is opposite to the catheter end 114, the control end 112 and the catheter end 114 are at different ends of the handpiece 110 and may face different directions relative to one another. In an embodiment, both the catheter 104 and the handpiece 110 are disposable. In some embodiments, the catheter 104 and the handpiece 110 are manufactured as an integral part, or the catheter 104 is fixed with the handpiece 110 via a handpiece-catheter connector 103. Alternatively, only the catheter 104 is disposable, and the handpiece 110 may be sterilized and reused multiple times. In this case, the catheter 104 is removably connected to the handpiece 110 and the catheter end 114 via a handpiece-catheter connector 103.

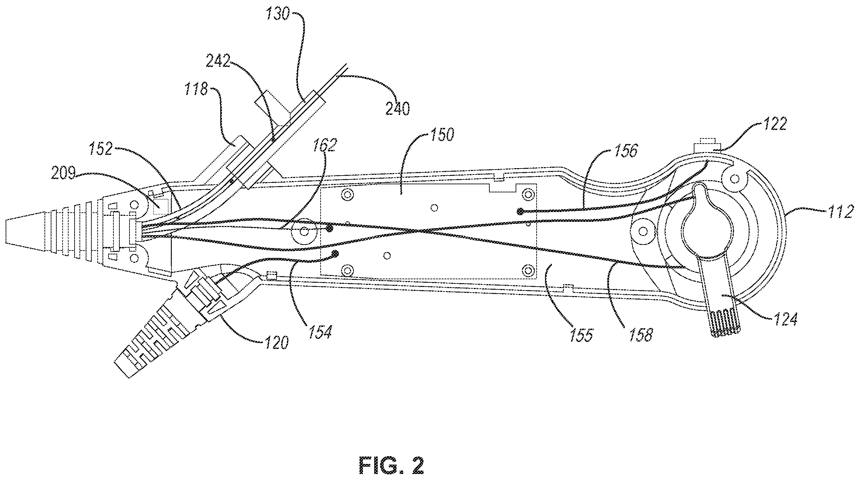

The catheter 104 of the ureteroscope 100 may be used for imaging an interior surface of a tubular structure, such as a lumen in the body of human or animal. For example, the catheter 104 may be inserted via a subject's urethra to access various parts of the urinary tract. However, it should be appreciated that the ureteroscope 100 may be employed as an industrial endoscope when tubular structure is a part of an industrial apparatus, an equipment, a product, a machine, a production line, and the like. In some embodiments, the catheter 104 may serve as a tether, and may include a plurality of scale markings or fiducials that enable a physician to measure a distance traveled by optoelectronic module into the tubular structure, such as a lumen of a body. Other structure(s) may be built into the ureteroscope 100 as desired. For example, the handpiece 110 may include a steering controller 124 configured to control one or more steering wires 158 (FIG. 2) that are connected to an active bend portion of the catheter 104 to deflect the distal end 105 to the desired location. Accordingly, a user may bend or curve the catheter 102 by moving the steering controller 124 on the handpiece 110.

The ureteroscope 100 may include an optoelectronic module (e.g., a camera or other imager) for imaging the interior of the subject. For example, turning to FIG. 1B, an optoelectronic module 109 (*e.g.,* image sensor) and at least one light source 111 may be located in a distal end 105 of the catheter 104 or other location in the catheter 104. The optoelectronic module 109 may include a micro camera module having an image sensor microchip, a set of micro lenses, and a micro illumination module. Suitable optoelectronic modules are disclosed in U.S. Patent No. 9,942,452, which is incorporated herein, in its entirety, by this reference. In some embodiments, the optoelectronic module 109 may be positioned in a rigid or semi-rigid shell-like housing at the distal end 105 configured for insertion into the tubular structure for imaging its interior surface. For example, the optoelectronic module 109 may be inserted into a patient's body through a natural body orifice, such as the mouth, nose, urethra, bladder, vagina, or anus. The ureteroscope 100 may therefore have different configurations for use as a gastrointestinal, a colonoscope, endoscopic ultrasound (EUS), endoscopic retrograde cholangiopancreatography (ERCP), or other suitable application. Applications of the ureteroscope 100 include diagnostic observation associated with endometrial polyps, infertility, abnormal bleeding, and pelvic pain, and surgical procedure such as embryo growth arrest and uterine malformation etc.

The ureteroscope 100 may include a receiving device or communication interface 150 **(****FIG. 2****)** generally located outside the catheter 104 for receiving the signal from an image sensor within the optoelectronic module 109. For example, the catheter 104 may include at least one electrical lead 162 **(****FIG. 2****)** that is coupled to the optoelectronic module 109 and conveys an electrical signal from optoelectronic module 109 to the communication interface 150. The communication interface 150 may be positioned within an interior region 155 **(****FIG. 2****)** of the handpiece 110 or adjacent to the handpiece 110.

The catheter 104 may be configured to couple the optoelectronic module 109 to the circuitry within the handpiece 110 in any suitable manner. For example, the availability of low-cost modular imaging system components enables the manufacture of a disposable components of the ureteroscope 100 at very low cost. In an embodiment, the catheter 104 is configured to detachably couple the optoelectronic module 109 to circuitry within the handpiece 110. In this manner, the catheter 104 and the optoelectronic module 109 are disposable, and may be detached from the handpiece 110 after a single patient use, thus eliminating the need for sterilization or reprocessing and reducing contamination risks. The handpiece 110 may be disinfected for subsequent reuse with a catheter 104 and optoelectronic module 109 for a different patient.

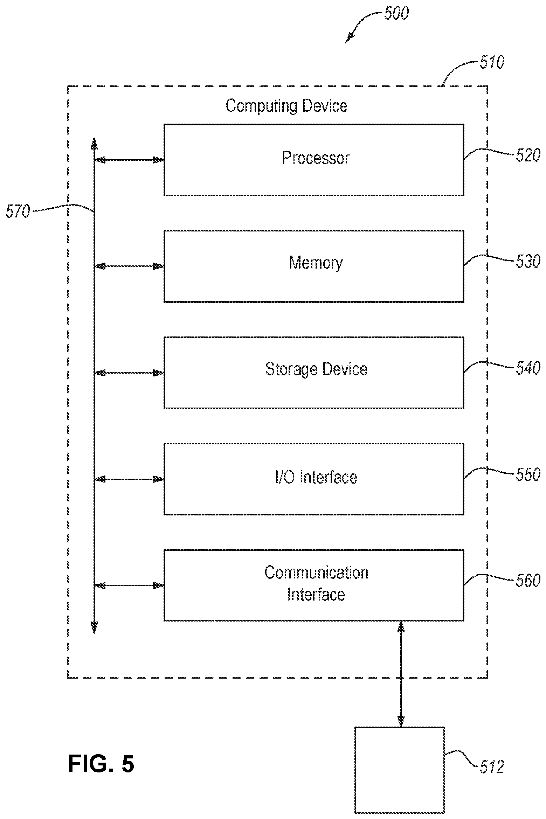

Returning to **FIG. 1A****,** the endoscopic system 10 may include one or more electronic devices for processing and displaying the image data received from the optoelectronic module 109 of the ureteroscope 100. For example, the endoscopic system 10 may include one or more of a host machine 170 having a microprocessor, a computer 180 having a microprocessor, and a display 190. Specific components of the host machine 170, the computer 180, and/or the display 190 are provided below in reference to **FIG. 5****.** The host machine 170 may be connected to one or more terminals of the computer 180 and the display 190 for further processing and displaying the image data from the optoelectronic module 109. The host machine 170 or the computer 180 may be programmed with image processing software that takes as input the image data output from the optoelectronic module 109 of the ureteroscope 100 and generates two- or three-dimensional reconstructions of the body lumen that may be displayed on the display 190. Accordingly, a processor in at least one of the host machine 170, the computer 180, or the display 190 may be programmed with software that accepts as input a plurality of still images of an object generated by the optoelectronic module 109, and then output for display a three-dimensional rendering of the object based on the plurality of still images. The display 190 may include any suitable display, and may be configured to display a moving image (*e.g.,* movie or video) or a still image collected by the image sensor of the optoelectronic module 109. Although shown in **FIG. 1A** as separate blocks, the host machine 170, the computer 180, and the display 190 may include a single device, two devices, three devices, or more than three devices.

The endoscopic system also may include a cable 102 configured to operably couple the ureteroscope to at least one of the host machine 170, the computer 180, or the display 190. The cable 102 may electrically couple the retrieving device 150 **(****FIG. 2****)** of the ureteroscope 100 to at least one of the host machine 170, the computer 180, or the display 190. The cable 102 also may allow the communication interface 150 to communicate with and receive electric power from the host machine 170 or other power sources. The cable 102 also may be configured to allow the communication interface 150 to transmit image data captured at the optoelectronic module 109 to the host machine 170 for processing, storing, and displaying.

Turning to **FIG. 2****,** which illustrates a side view of the handpiece 110 with a portion of the handpiece 110, the ureteroscope 100 may include a communication interface 150 or host interface housed in the handpiece 110. The communication interface 150 may contain, for example, one or more of a processor board, a camera board and frame grabber, or a power source. The processor board may be coupled by the cable 102 to the host machine 170 for storage and retrieval of images generated by ureteroscope 100. The communication interface 150 also may be configured to communicate with and receive electric power from the host machine 170 or other power source via the cable 102. The communication interface 150 also may transmit image data captured at the distal end 105 to the host machine 170 for processing, storing, and displaying. The communication interface 150 may be connected to the cable 102 through one or more wires 154 and/or connected to the optoelectronic module 109 through one or more electrical leads 162.

Alternatively or in addition, the communication interface 150 or handpiece 110 may include an antenna and a wireless chipset, e.g., compliant with the IEEE 802.11 Wi-Fi standards, for wirelessly transmitting the video or still image generated by the ureteroscope 100 to the host machine 170, the computer 180, or the display 190 without the cable 102. For example, the communication interface 150 may include a wireless interface configured to implement various protocols, including but not limited to Wi-Fi, Bluetooth, ZigBee, Z-Wave, etc. This arrangement may be useful in a physician's office because it permits the computer and display to be placed outside of the sterile field, while also allowing the physician greater maneuverability during use of the ureteroscope 100. In other embodiments, the communication interface 150 may be omitted and the image data may be transmitted directly to the host machine 170 and/or the computer 180.

The ureteroscope 100 may include one or more controls 122 positioned at or proximate to the control end 112 of the handpiece 110. The one or more controls 122 may include one or more of a switch, a button, a rotatable knob, a movable tab, and the like. The one or more controls may be electrically coupled to the communication interface 150 through one or more wires 156. In some embodiments, the one or more controls 122 are configured to adjust views presented on the display 190. For example, the one or more controls may be configured to adjust at least one of a brightness, a zoom, a focus or a contrast of one or more images displayed on the display 190. Accordingly, the one or more controls 122 allow a user to adjust views presented on the display 190 and/or computer 180 according to the user's preference and as necessary during use of the ureteroscope 100.

The ureteroscope 100 may include one or more wires coupling the one or more controls 122 to the optoelectronic module 109 to allow a user to adjust at least one of the brightness, the zoom, the focus, or the contrast on the optoelectronic module 109. For example, at least one of the one or more controls 122 may be connected to the optoelectronic module 109 through one or more wires 156 connected to the communication interface 150 and at least one of the one or more control 122 and an electric lead 162 connected to the communication interface 150 and the optoelectronic module 109. In some embodiments, one or more wires or electric leads may be connected directly to the optoelectronic module 109 and at least one of the one or more control 122. Coupling the one or more controls 122 to the communication interface 150 may provide the technical effect of allowing a user to activate the laser fiber and/or adjust at least one of the brightness, the zoom, the focus, or the contrast on the display 190, either wirelessly or through the cable 102.

**In** some embodiments, at least one of the one or more controls 122 is configured to activate *(e.g.,* turn on) or deactivate *(e.g.,* turn off) at least one light source 111 **(****FIG. 1B****)** at the distal end 105 of the catheter 104. For example, the light source 111 may include a light-emitting diode (LED) positioned in the distal end 105 of the catheter. Alternatively or in addition, the ureteroscope 100 may include a LED light source positioned elsewhere, such as in the handpiece 110, which provides illumination to the at least one light source 111 at the distal end 105 via one or more sets of optical fibers extending through the catheter 104. In some instances of use, it is desirable to turn the at least one light source 111 of the ureteroscope 100 completely off during backloading of the ureteroscope 100 over guidewires, rather than merely incrementally adjusting the light. Accordingly, at least one of the one or more controls allows a user to selectively turn off and turn on the light source 111 when desired by the user.

**In** some embodiments, at least one of the one or more controls 122 is configured to activate and deactivate the optoelectronic module 109. At least one of the one or more controls 122 also may be configured to switch an image mode in at least one of the optoelectronic module 109, the retrieving device 150, or the one or more electronic devices between a still image mode whereby a still image is recorded and a video image mode whereby a video stream is recorded. At least one of the one or more controls 122 also may be configured to activate a frame grabber mode in at least one of the optoelectronic module 109, the retrieving device 150, or the one or more electronic devices that creates a still image from the video stream output generated by the optoelectronic module 109. For example, at least one of the one or more controls 122 may be configured to communicate with one or more of the host machine 170, the computer 180, or the display 190 to allow a user to create a still image from a video stream being displayed on the display 190, and storing or recording the still image on at least one of the host machine 170 or the computer 180.

**In** some embodiments, at least one of the one or more controls 122 is configured to activate and/or deactivate the laser fiber 140. At least one of the one or more controls 122 may be configured to activate the laser fiber 140 such that the emitting end 142 of the laser fiber 140 emits laser light. At least one of the one or more controls 122 may be configured to deactivate the laser fiber 140 such that the emitting end 142 of the laser fiber 140 ceases to emit laser light.

The ureteroscope 100 may be operated to perform or complete selected tasks manually, automatically, or a combination thereof. Some ureteroscopic functions may be implemented with the use of components that comprise hardware, software, firmware or combinations thereof. While general-purpose components such as general purpose computers or oscilloscopes may be used in the ureteroscope 100, dedicated or custom components such as circuits, integrated circuits or software may be too. For example, some functions are implemented with a plurality of software instructions executed by one or more data processors, which is part of a general-purpose or custom computer. The one or more data processors may be in at least one of the communication interface 150, the host machine 170, the computer 180, or the display 190. In some embodiments, the data processor or computer comprises volatile memory for storing instructions and/or data and/or a non-volatile storage, for example, a magnetic hard-disk and/or removable media, for storing instructions and/or data. In some embodiments, implementation includes a network connection. In some embodiments, implementation includes a user interface, generally comprising one or more input devices (e.g., allowing input of commands and/or parameters) and output devices (e.g., allowing reporting parameters of operation and results).

The handpiece 110 also includes a working channel port 118 and a working channel opening 107 **(****FIG. 1B****)** that provides fluid communication between the working channel port 118 and the distal end 105 of the catheter 104. The handpiece 110 also may include a working channel connector 130 that is connected or attached to the working channel port 118 and a working channel 152 **(****FIG. 2****)** that provides fluid communication between working channel port 118 and the working channel opening 107 through the handpiece 110 and/or the catheter 104. The working channel 152 may include a single, continuous material through the handpiece 110 and the catheter 104. In some embodiments, the working channel 152 includes a first portion in the interior region 155 of the handpiece 110 and a second portion in the catheter 104. The first portion and the second portion of the catheter 152 may include different materials or configurations that are fluidly connected to one another. At least one of the working channel port 118 or the working channel connector 130 is configured to engage with various surgical instruments and irrigation devices such as the laser fiber 140, as needed, for operations such as stone breaking and retrieval, etc. The working channel port 118 may be positioned on a lower/bottom or an upper/top portion of the handpiece 110, proximate to the catheter end 114 of the handpiece 110. The working channel port 118 may be positioned less than one-half of a distance from the catheter end 114 to the control end 112, less than one-third of the distance from the catheter end 114 to the control end 112, less than one-quarter of the distance from the catheter end 114 to the control end 112, or less than one-fifth of the distance from the catheter end 114 to control end 112.

The handpiece 110 also may include a cable port 120 for receiving or connecting the cable 102 **(****FIGS. 1A****),** thereby operably coupling the ureteroscope 100 to at least one of the host machine 170, the computer 180, or the display 190. Both the cable port 120 and the working channel port 118 also may be positioned on a lower or bottom portion of the handpiece 110, proximate to the catheter end 114 of the handpiece 110. For example, both the cable port 120 and the working channel port 118 may be positioned less than one-half of a distance from the catheter end 114 to the control end 112, less than one-third of the distance from the catheter end 114 to the control end 112, less than one-quarter of the distance from the catheter end 114 to the control end 112, or less than one-fifth of the distance from the catheter end 114 to control end 112. Alternatively, the handpiece 110 may further include a compact battery module for supplying power to the optoelectronic module 109 and the at least one light source 111. The power source in the handpiece 110 may be, for example, one or more conventional dry-cell disposable batteries or lithium ion rechargeable batteries.

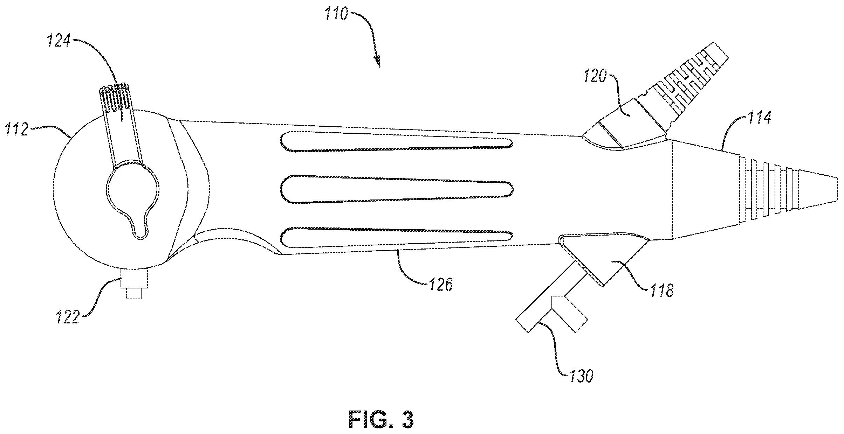

Features from any of the disclosed embodiments may be used in combination with one another, without limitation. For example, while the ureteroscope 100 shown in **FIGS. 1-3** includes one or more controls 122 positioned at the control end 112 of the handpiece 110, in some embodiments, the one or more controls 122 are absent from the handpiece 110. Accordingly, in some embodiments, the one or more controls 122 are absent from the handpiece 110, but the handpiece 110 includes a cable port 120 positioned proximate to the catheter end 114, a cable 102 connected or connectable to the handpiece 110 at the cable port 118, and a working channel port 118 disposed on the rounded first surface 126 along the longitudinal central plane proximate to the catheter end 114. The cable 102 may be directly or indirectly connected or coupled to the handpiece 110 at the cable port 118.

Moreover, while the ureteroscope 100 shown in **FIGS. 1-3** includes a cable port 120 positioned proximate to the catheter end 114 and distal to the control end 112, in some embodiments, the cable port 120 is absent from the handpiece 110 or positioned proximate to the control end 112 and distal to the catheter end 114. Accordingly, in some embodiments, the cable port 120 is absent from the handpiece 110 or positioned proximate to the control end 112, but the handpiece 110 includes one or more controls 122 positioned at the control end 112 of the handpiece 110 and a working channel port 118 disposed on the rounded first surface 126 along a longitudinal central plane proximate to the catheter end 114. In still other embodiments, the one or more controls 122 are absent from the handpiece 110 and the cable port 120 is absent from the handpiece 110 or positioned proximate to the control end 112, but the handpiece includes a working channel port 118 disposed on the rounded first surface 126 along the longitudinal central plane proximate to the catheter end 114.

Furthermore, while the ureteroscope 100 shown in **FIGS. 1-3** includes a working channel port 118 disposed on the rounded first surface 126 along the longitudinal central plane proximate to the catheter end 114 and distal to the control end 112, in some embodiments, the working channel port 118 may be disposed elsewhere on the handpiece 110. Accordingly, in some embodiments, the working channel port 118 may be disposed elsewhere than along the longitudinal central plane proximate to the catheter end 114, but the handpiece may include one or more controls 122 positioned at the control end 112 of the handpiece 110 and a cable port 120 positioned proximate to the catheter end 114. In still other embodiments, the one or more controls 122 are absent from the handpiece 110 and the working channel port 118 may be disposed elsewhere than along the longitudinal central plane proximate to the catheter end 114, but the handpiece 110 includes a cable port 120 positioned proximate to the catheter end 114. In some embodiments, the cable port 120 is absent from the handpiece 110 or positioned proximate to the control end 112 and the working channel port 118 may be disposed elsewhere than along the longitudinal central plane proximate to the catheter end 114, but the handpiece 110 includes the one or more controls 122 positioned at the control end 112 of the handpiece 110.

Returning to **FIG. 1A****,** a laser fiber 140 may be used with the endoscopic system 10. The laser fiber 140 includes an emitting end 142 configured to selectively emit a laser light that fragments stones in the body of a subject. For example, the laser fiber 140 may be used with the endoscopic system 10 during surgical procedure known as a laser lithotripsy to remove stones from the urinary tract of a subject. In some embodiments, the laser fiber 140 and the endoscopic system 10 may be used to remove stone from the kidney, ureter, bladder, and/or urethra of the subject. During the laser lithotripsy procedure, the catheter 104 of the ureteroscope 100 is inserted into the urinary tract of the subject to locate the stone. The laser fiber 140 is inserted into the working channel connector 130 and the working channel port 118, through the working channel 152 of the ureteroscope and out of the working channel opening 107. For example, the laser fiber 140 may be manually fed *(e.g.* hand fed) through the working channel 152. Laser light may be emitted from the emitting end 142 of the laser fiber 140 to fragment or break apart the stone. The stone is fragmented by the laser light, and the remaining pieces may be collected in a basket and/or washed out of the urinary tract of the subject.

The laser fiber 140 may include any of a variety of laser fibers known in the art. For example, the laser fiber 140 may include a silica material and various wavelengths. The laser fiber 140 may include a diameter of about 200 microns (or micrometers) to about 1000 microns. **In** some embodiments, the laser fiber 140 may include a pulsed dye laser, a Ho:YAG laser, a Nd:YAG laser, a holmium laser, or a thulium fiber laser. For example, the laser fiber may include a single-use fiber configured to deliver focused holmium energy. The laser fiber 140 may include a jacket 144 providing an outer protective surface or covering for at least a portion of the laser fiber 140. The emitting end 142 of the laser fiber 140 may be spaced from the terminating end of the jacket 144. The laser fiber 140 may be powered separately from the endoscopic system 10 or may be electrically coupled to and powered by the endoscopic system 10.

During use of the laser fiber 140 with the endoscopic system 10, the laser light emitted from the emitting end 142 of the laser fiber 140 can damage the ureteroscope 100 if the laser light is emitted while the emitting end 142 of the laser fiber 140 is still in the working channel 152 of the ureteroscope 100. The configuration of the endoscopic system 10 may provide the technical effect of preventing or inhibiting the laser light from emitting (*e.g.,* firing) from the emitting end 142 while the emitting end 142 of the laser fiber is still in the working channel 152 of the ureteroscope 100. For example, in some embodiments, the endoscopic system 10 includes one or more sensors positioned to detect when at least a portion of a laser fiber 140 (1) enters the working channel 152, (2) passes through the working channel 152, and/or (3) has exited the working channel 152 through the working channel opening 107 in the distal end 105 of the catheter 104. A processor in at least one of the host machine 170, the computer 180, and/or the display 190 that is in communication with the one or more sensors may be configured to determine if the emitting end 142 of the laser fiber 140 is outside the working channel 152 and spaced at least a predetermined distance from the distal end 105 of the catheter 104 such that emitting laser light from the emitting end 142 of the laser fiber 140 does not damage the catheter 104 and/or the endoscope 100. Thus, the configuration of the endoscopic system 10 results in the technical effect of preventing or inhibiting damage to the ureteroscope 100 and/or the laser fiber 140 by ensuring the laser light is emitted from the emitting end 142 of the laser fiber only when the emitting end 142 is outside the working channel 152 and spaced a predetermined distance from the distal end 105 of the catheter.

**In** some embodiments, a processor in at least one of the host machine 170, the computer 180, and/or the display 190 uses data collected from the optoelectronic module 109 *(e.g.,* image sensor) at the distal end 105 of the catheter 104 to determine if the emitting end 142 of the laser fiber 140 is out of working channel 152 and spaced a predetermined distance from the distal end 105 of the catheter 104. For example, the processor in the host machine 170 and/or the computer 180 may determine or otherwise measure a color of the laser fiber (*e.g.,* blue) on the display 190 that is displaying images or video from the optoelectronic module 109. When the processor determines enough of the color of the laser fiber 140 is visible on the display 190 to indicate that the emitting end 142 of the laser fiber 140 is outside the working channel 152 and spaced the predetermined distance from distal end 105 of the catheter 140, an alert may be displayed or otherwise signaled indicating that it is safe to fire or emit the laser light from the emitting end 142 of the laser fiber 140.

**In** some embodiments, the endoscopic system includes at least the image sensor of the optoelectronic module 109 positioned at the distal end 105 of the catheter 104. The image sensor of the optoelectronic module is configured to detect at least a portion of the laser fiber 140 has exited the working channel 152 through the working channel opening 107 in the distal end of the catheter 104. The processor is configured to determine if the emitting end 142 of the laser fiber 140 is outside the working channel 152 and spaced at least the predetermined distance from the distal end 105 of the catheter 104 based on data from the image sensor of the optoelectronic module 109. In some embodiments, the predetermined distance is spacing the emitting end 142 at least about 3 mm to about 8 mm from the distal end 105 of the catheter 104. Spacing the emitting end 142 at least 3 mm to about 8 mm from the distal end 105 of the catheter 104 and outside the working channel 152 provides the technical effect of preventing or inhibiting the laser light from damaging the catheter 104 and/or the handpiece when the emitting end 142 emits the laser light.

**In** some embodiments, the endoscopic system 10 is configured to display, in real time, images (*e.g.,* still images, movie or video images) collected from the image sensor of the optoelectronic module 109 on the display 190. The processor in at least one of the host machine 170, the computer 180, and/or the display 190 is configured to determine if the emitting end 142 of the laser fiber 140 is outside the working channel 152 and spaced at least the predetermined distance from the distal end 104 of the catheter 104. For example, the processor may determine an amount of the laser fiber 140 visible on the display 190 displaying in real time the images collected from the image sensor of the optoelectronic module. In some embodiments, the processor may be configured to determine if the emitting end 142 of the laser fiber 140 is outside the working channel 152 and spaced at least the predetermined distance from the distal end 105 of the catheter 104 by determining a number or ratio of pixels on the display 190 displaying a color of the laser fiber 140. For example, the laser fiber 140 (*e.g.,* the fiber jacket 144) may include a blue color. The processor may be configured to measure or determine the number or ratio of pixels on the display 190 that are displaying the blue color of the laser fiber 140. A predetermined number or ratio or pixels on the display 190 may indicate that the emitting end 142 of the laser fiber 140 is outside the working channel 152 and spaced at least the predetermined distance from the distal end 105 of the catheter 104. In other embodiments, the laser fiber 140 may include other colors (*e.g.,* red, green, yellow, etc.) similarly detected by the image sensor of the optoelectronic module 109 and used to determine if the emitting end 142 of the laser fiber 140 is outside the working channel 152 and spaced at least the predetermined distance from the distal end 105 of the catheter 104. In some embodiments, the processor in at least one of the host machine 170, the computer 180, and/or the display 190 is configured to use data from the image sensor of the optoelectronic module 109 to determine the emitting end 142 of the laser fiber 140 is outside the working channel 152 and spaced at least the predetermined distance from the distal end 105 of the catheter 104 by using an algorithm that uses data from the image sensor in the optoelectronic module 109 to calculate a distance for the emitting end 142 of the laser fiber 140 from the optoelectronic module 109.

The endoscopic system 10 also may be configured to provide an alert to indicate when the emitting end 142 is or is not outside the working channel 152 and/or spaced the predetermined distance from the distal end 105 of the catheter 104. For example, the processor in at least one of the host machine 170, the computer 180, and/or the display 190 may be configured to coordinate an alert on the display 190 if the emitting end 142 of the laser fiber 140 is outside the working channel 152 and spaced at least the predetermined distance from the distal end 105 of the catheter 104. For example, the display 190 may display a green box or icon when the emitting end 142 of the laser fiber 140 is outside the working channel 152 and spaced at least the predetermined distance from the distal end 105 of the catheter 104. The processor in at least one of the host machine 170, the computer 180, and/or the display 190 may be configured to coordinate an alert on the display 190 if the emitting end 142 of the laser fiber 140 is not outside the working channel 152 and/or not spaced at least the predetermined distance from the distal end 105 of the catheter 104. For example, the display 190 may display a red box or icon when the emitting end 142 of the laser fiber 140 is not outside the working channel 152 *(e.g.,* still inside the working channel 152) and/or not spaced at least the predetermined distance from the distal end 105 of the catheter 104. In some embodiments, the configuration of the endoscopic system 10 provides the technical effect of preventing or inhibiting the emitting end 142 of the laser fiber from emitting the laser light until the processor determines the emitting end 142 of the laser fiber 140 is outside the working channel 152 and spaced at least the predetermined distance from the distal end 105 of the catheter 104. In some embodiments, the configuration of the endoscopic system 10 results in the technical effect of preventing the emitting end 142 of the laser fiber from emitting the laser light when the emitting end 142 is inside the working channel 152.

**In** some embodiments, the endoscopic system 10 may include a sensor positioned inside or proximate to the handpiece 110 of the ureteroscope 100 to detect the laser fiber 140 passing through the working channel 152, the working channel port 118, and/or the working channel connector 130. The laser fiber 140 and/or the jacket 144 on the laser fiber 140 may include one or more markers (e.g., dots, lines, or barcodes). The sensor may be positioned to detect the one or more markers and a processor associated with the sensor may interpret and/or count the markers passing through the working channel 152 and determine when the emitting end 142 of the laser fiber 140 is outside the working channel 152 and spaced the predetermined distance from the distal end 105 of the catheter 104.

Turning to **FIG. 2****,** the endoscopic system 10 includes at least a sensor 209 positioned to detect the laser fiber 140 or the jacket 144 of the laser fiber 140 passing through the working channel 152. The sensor 209 may include an image sensor, according to some embodiments. In some embodiments, the sensor 209 may include a metal or magnet sensor. The sensor 209 may be electrically coupled or in communication with the processor of at least one of the host machine 170, the computer 180, and/or the display 190. In some embodiments, the endoscopic system 10 does not utilize data from the image sensor of the optoelectronic module 109, but instead uses data from the sensor 209 to determine if the emitting end 142 of the laser fiber 140 is outside the working channel 152 and spaced the predetermined distance from the distal end 105 of the catheter 104. In some embodiments, the sensor 209 is absent from the endoscopic system 10, and the endoscopic system 10 uses data from the image sensor of the optoelectronic module 109 to determine if the emitting end 142 of the laser fiber 140 is outside the working channel 152 and spaced the predetermined distance from the distal end 105 of the catheter 104, as described above.

At least some of the first portion of the working channel 152 passing through the interior region 155 of the handpiece 110 may be translucent or transparent. The sensor 209 is positioned in the interior region 155 of the handpiece 110 at least proximate to the first portion of the working channel 152 that is translucent or transparent. The sensor 209 is configured to detect the laser fiber 140 and/or the jacket 144 of the laser fiber 140 passing through the first portion of the working channel 152 that is translucent or transparent. In some embodiments, processor in at least one of the host machine 170, the computer 180, and/or the display 190 is configured to determine if the emitting end 142 of the laser fiber 140 is outside the working channel 152 and spaced at least the predetermined distance from the distal end 105 of the catheter 104 based on the sensor 209 detecting the emitting end 142 and a predetermined length of the laser fiber 140 or the jacket 144 of the laser fiber 140 passing through the first portion of the working channel 152 that is translucent or transparent.

In some embodiments, the jacket 144 of the laser fiber 140 includes a plurality of markers 242 on the jacket 144. The sensor 209 is configured to detect the plurality of markers 242 and the processor in at least one of the host machine 170, the computer 180, and/or the display 180 is configured to determine if the emitting end 142 of the laser fiber 140 is outside the working channel 152 and spaced at least the predetermined distance from the distal end 105 of the catheter 104 based on at least a predetermined number of markers of the plurality of markers 242 passing and detected by the sensor 209. The markers may include dots, barcodes, and/or material detectable by the sensor 209 that is different from the rest of the jacket 144. For example, the markers may be spaced at a predetermined interval from one another, such as about 1.5 cm to about 2 cm between each marker of the plurality of markers 242. The processor may be configured to count how many of the markers have been detected by the sensor 209 and, when a predetermined number of markers of the plurality of markers 242 have been detected by the sensor, indicate the emitting end 142 of the laser fibers is outside the working channel 152 and spaced at least the predetermined distance from the distal end 105 of the catheter 104. In some embodiments, the plurality of markers 242 may include one or more barcodes, fiducial marks, magnetic markers, and/or metal markers readable or detectable by the sensor 209 and the processor may be configured to use that data from the sensor reading the one or more barcodes on the fiber jacket 144 to determine how much of the laser fiber 140 has passed the sensor 209 and/or if the emitting end 142 of the laser fibers is outside the working channel 152 and spaced at least the predetermined distance from the distal end 105 of the catheter 104. In some embodiments, the endoscopic system 10 is configured to coordinate an alert on the display 190 (1) if the emitting end 142 of the laser fiber 140 is outside the working channel 152 and spaced at least the predetermined distance from the distal end 105 of the catheter 104 and/or (2) if the emitting end 142 of the laser fiber 140 is not outside the working channel 150 and/or not spaced at least the predetermined distance from the distal end 105 of the catheter 104.

Although shown in the interior region 155 of the handpiece 110 in **FIG. 2****,** in some embodiments the sensor 209 may positioned on the handpiece 110 proximate to the working channel port 118 and/or the working channel connector 130. For example, the sensor 209 may be positioned on the outside of the handpiece 110. In these and other embodiments, the processor may be configured to determine if the emitting end 142 of the laser fiber 140 is outside the working channel 152 and spaced at least the predetermined distance from the distal end 104 of the catheter 105 based on the sensor 209 detecting the emitting end 142 and a predetermined length of the laser fiber 140 or the jacket 144 of the laser fiber 140 entering the working channel 152 through the working channel port 118 and/or the working channel connector 130. In these and other embodiments, the jacket 144 may include the plurality of markers 242. The sensor 209 may be configured to detect the plurality of markers and the processor may be configured to determine if the emitting end 142 of the laser fiber 140 is outside the working channel 152 and spaced at least the predetermined distance from the distal end 105 of the catheter 104 based on at least a predetermined number of markers of the plurality of markers 242 entering the working channel 152 through the working channel port 118 or the working channel connector 130.

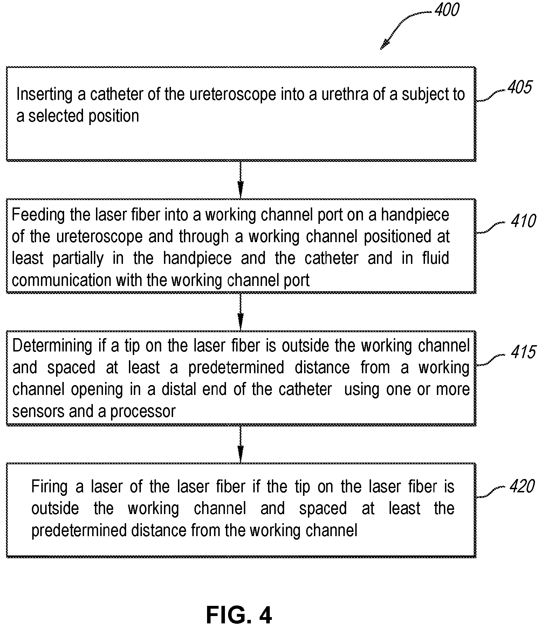

**FIG. 4** is a flow diagram of a method 400 of using a laser fiber with a ureteroscope, according to an embodiment. The method 400 may include use of any embodiment of the endoscopic system 10 described above. The method includes an act 405 of inserting a catheter of the ureteroscope into a urethra of a subject to a selected position. The method 400 includes an act 410 of feeding (e.g., inserting) the laser fiber into a working channel port on a handpiece of the ureteroscope and directing (e.g. pushing) the laser fiber through a working channel positioned at least partially in the handpiece and the catheter and in fluid communication with the working channel port. The method 400 includes an act 415 of determining if an emitting end on the laser fiber is outside the working channel and spaced at least a predetermined distance from a working channel opening in a distal end of the catheter distal to the handpiece using one or more sensors and a processor in communication with the one or more sensors. The method 400 includes an act 420 of emitting a laser light from the emitting end of the laser fiber if the emitting end on the laser fiber is determined to be outside the working channel and spaced at least the predetermined distance from the working channel. The laser light emitted from the emitting end of the laser fiber fragments or breaks apart stones proximate to the emitting end of the laser light. In some embodiments, the method 400 may optionally include an act of feeding *(e.g.* pushing) the laser fiber further through the working channel if the emitting end of the laser fiber is determined to not be outside the working channel and/or is not spaced at least the predetermined distance from the working channel. The method 400 may then return to the acts 415 and 420.

In some embodiments, the act 415 includes determining if the emitting end on the laser fiber is outside the working channel and spaced at least the predetermined distance from the working channel opening using the processor and data from an image sensor positioned at the distal end of the catheter that detects at least a portion of the laser fiber has exited the working channel through the working channel opening. In these and other embodiments, the method 400 may include an act of displaying in real time images collected from the image sensor on a display in communication with the processor. The act 415 may then further include determining, with the processor, if the emitting end on the laser fiber is outside the working channel and spaced at least the predetermined distance from the working channel opening by determining an amount of the laser fiber visible on the display displaying in real time the images collected from the image sensor. More particularly, the act 415 may include determining, with the processor, if the emitting end of the laser fiber is spaced at least the predetermined distance from the distal end by determining a number or ratio of pixels on the display displaying a color of the laser fiber. In these and other embodiments, the method 400 may further include coordinating, with the processor, an alert on the display if the emitting end of the laser fiber is outside the working channel spaced at least the predetermined distance from the distal end of the catheter. The method 400 may include coordinating, with the processor, an alert on the display if the emitting end of the laser fiber is not outside the working channel and/or spaced at least the predetermined distance from the distal end of the catheter.

In some embodiments, the act 415 may include (1) an act of detecting the laser fiber or a j acket on the laser fiber entering the working channel port and/or passing through the working channel with at least one sensor positioned proximate to the working channel port and/or the working channel and (2) an additional act of determining if the emitting end on the laser fiber is outside the working channel and spaced at least the predetermined distance from a working channel opening using the processor and data from the at least one sensor detecting the laser fiber or the jacket on the laser fiber. In these and other embodiments, the act of detecting the laser fiber or a jacket on the laser fiber entering the working channel port and/or passing through the working channel with at least one sensor positioned proximate to the working channel port and/or the working channel may include detecting the laser fiber or the jacket on the laser fiber passing through a portion of the working channel that is translucent or transparent with the at least one sensor that is positioned in the interior region of the handpiece at least proximate to the portion of the working channel that is translucent or transparent.

More particularly, the act of detecting the laser fiber or the jacket on the laser fiber passing through a portion of the working channel that is translucent or transparent with the at least one sensor that is positioned in the interior region of the handpiece at least proximate to the portion of the working channel that is translucent or transparent may include detecting the emitting end and a predetermined length of the laser fiber or the jacket on the laser fiber passing through the portion of the working channel that is translucent or transparent with the at least one sensor that is positioned in the interior region of the handpiece at least proximate to the portion of the working channel that is translucent or transparent. In these and other embodiments, the act of determining if the emitting end on the laser fiber is outside the working channel and spaced at least the predetermined distance from a working channel opening using the processor and data from the at least one sensor detecting the laser fiber or the jacket on the laser fiber may include determining, using the processor, if the emitting end of the laser fiber is spaced at least the predetermined distance from the distal end of the catheter based on data form the at least one sensor detecting the emitting end and the predetermined length of the laser fiber or the jacket on the laser fiber passing through the portion of the working channel that is translucent or transparent.

In some embodiments, the act of detecting the laser fiber or the jacket on the laser fiber passing through a portion of the working channel that is translucent or transparent with the at least one sensor that is positioned in the interior region of the handpiece at least proximate to the portion of the working channel that is translucent or transparent may include detecting a predetermined number of markers of a plurality of markers on the laser fiber or the jacket on the laser fiber passing through a portion of the working channel that is translucent or transparent with the at least one sensor that is positioned in the interior region of the handpiece at least proximate to the portion of the working channel that is translucent or transparent. In these and other embodiments, the act of determining if the emitting end on the laser fiber is outside the working channel and spaced at least the predetermined distance from a working channel opening using the processor and data from the at least one sensor detecting the laser fiber or the jacket on the laser fiber may include determining if the emitting end on the laser fiber is outside the working channel and spaced at least the predetermined distance from a working channel opening using the processor and data from the at least one sensor of the predetermined number of markers of the plurality of markers on the laser fiber or the jacket on the laser fiber passing through a portion of the working channel.

**In** some embodiments of the method 400 described above, the method 400 further includes coordinating, with the processor, an alert on a display if the emitting end of the laser fiber is outside the working channel spaced at least the predetermined distance from the distal end of the catheter. The method 400 also may include an act of coordinating, with the processor, an alert on a display if the emitting end of the laser fiber is not outside the working channel and/or spaced at least the predetermined distance from the distal end of the catheter.

The acts of the method 400 described above, including but not limiting to the acts 405, 410, 415, and 420, are for illustrative purposes. For example, the acts of the method 400 may be performed in different orders, split into multiple acts, modified, supplemented, or combined. In an embodiment, one or more of the acts of the method 400 may be omitted from the method 400. Any of the acts of the method 900 may include using any of the handpieces 110, ureteroscopes 100, and/or the system 10 disclosed herein.

Systems and methods described herein may include use a controller to carry out one or more aspects of the systems and methods. For example, a controller may carry out the act 415 of the method 400. Moreover, one or more *(e.g.,* all) of the communication interface 150 in the handpiece 110, the host machine 170, the computer 180, and/or the display 190 of the endoscopic system 10 may utilize a controller. For example, the processor described in relation to the endoscopic system 10 may include or utilize a controller. **FIG. 5** is a schematic of a controller 500 that may be utilized in any of the example systems and method described herein, according to an embodiment. The controller 500 may be configured to implement one or more acts of any of the example methods disclosed herein, such as the act 415 of the method 400. The controller 500 includes at least one computing device 510. The at least one computing device 510 is an exemplary computing device that may be configured to perform one or more of the acts described above, such as the act 415 of the method 400. The at least one computing device 510 can include one or more servers, one or more computers (e.g., desk-top computer, laptop computer), or one or more mobile computing devices (e.g., smartphone, tablet, etc.). The computing device 510 can comprise at least one processor 520, memory 530, a storage device 540, an input/output ("I/O") device/interface 550, and a communication interface 560. While an example computing device 510 is shown in **FIG. 5****,** the components illustrated in **FIG. 5** are not intended to be limiting of the controller 500 or computing device 510. Additional or alternative components may be used in some examples. Further, in some examples, the controller 500 or the computing device 510 can include fewer components than those shown in **FIG. 5****.** For example, the controller 500 may not include the one or more additional computing devices 512. In some examples, the at least one computing device 510 may include a plurality of computing devices, such as a server farm, computational network, or cluster of computing devices. Components of computing device 510 shown in **FIG. 5** are described in additional detail below.

**In** some examples, the processor(s) 520 includes hardware for executing instructions (e.g., instructions for carrying out one or more portions of any of the methods disclosed herein), such as those making up a computer program. For example, to execute instructions, the processor(s) 520 may retrieve (or fetch) the instructions from an internal register, an internal cache, the memory 530, or a storage device 540 and decode and execute them. As an example, the processor(s) 520 may include one or more instruction caches, one or more data caches, and one or more translation lookaside buffers (TLBs). Instructions in the instruction caches may be copies of instructions in memory 530 or storage device 540. In some examples, the processor 520 may be configured (e.g., include programming stored thereon or executed thereby) to carry out one or more portions of any of the example methods disclosed herein.

**In** some examples, the processor 520 is configured to perform any of the acts disclosed herein such as the act 415 in the method 400 or cause one or more portions of the computing device 510 or controller 500 to perform at least one of the acts disclosed herein. Such configuration can include one or more operational programs (e.g., computer program products) that are executable by the at least one processor 520. For example, the processor 520 may be configured to automatically determine if an emitting end on the laser fiber is outside the working channel and spaced at least a predetermined distance from a working channel opening in a distal end of the catheter distal to the handpiece using one or more sensors and a processor in communication with the one or more sensors.

The at least one computing device 510 (e.g., a server) may include at least one memory storage medium (e.g., memory 530 and/or storage device 540). The computing device 510 may include memory 530, which is operably coupled to the processor(s) 520. The memory 530 may be used for storing data, metadata, and programs for execution by the processor(s) 520. The memory 530 may include one or more of volatile and non-volatile memories, such as Random Access Memory (RAM), Read Only Memory (ROM), a solid state disk (SSD), Flash, Phase Change Memory (PCM), or other types of data storage. The memory 530 may be internal or distributed memory.

The computing device 510 may include the storage device 540 having storage for storing data or instructions. The storage device 540 may be operably coupled to the at least one processor 520. In some examples, the storage device 540 can comprise a non-transitory memory storage medium, such as any of those described above. The storage device 540 (e.g., non-transitory storage medium) may include a hard disk drive (HDD), a floppy disk drive, flash memory, an optical disc, a magneto-optical disc, magnetic tape, or a Universal Serial Bus (USB) drive or a combination of two or more of these. Storage device 540 may include removable or non-removable (or fixed) media. Storage device 540 may be internal or external to the computing device 510. In some examples, storage device 540 may include non-volatile, solid-state memory. In some examples, storage device 540 may include read-only memory (ROM). Where appropriate, this ROM may be mask programmed ROM, programmable ROM (PROM), erasable PROM (EPROM), electrically erasable PROM (EEPROM), electrically alterable ROM (EAROM), or flash memory or a combination of two or more of these. In some examples, one or more portions of the memory 530 and/or storage device 540 (e.g., memory storage medium(s)) may store one or more databases thereon.

In some examples, data from the one or more sensors of the endoscopic system 10, for example, may be stored in a memory storage medium such as one or more of the at least one processor 520 (e.g., internal cache of the processor), memory 530, or the storage device 540. In some examples, the at least one processor 520 may be configured to access (e.g., via bus 570) the memory storage medium(s) such as one or more of the memory 530 or the storage device 540. For example, the at least one processor 520 may receive and store the data (e.g., look-up tables) as a plurality of data points in the memory storage medium(s). The at least one processor 520 may execute programming stored therein adapted access the data in the memory storage medium(s) to automatically determine if an emitting end on the laser fiber is outside the working channel and spaced at least a predetermined distance from a working channel opening in a distal end of the catheter distal to the handpiece using one or more sensors and a processor in communication with the one or more sensors.

The computing device 510 also includes one or more I/O devices/interfaces 550, which are provided to allow a user to provide input to, receive output from, and otherwise transfer data to and from the computing device 510. These I/O devices/interfaces 550 may include a mouse, keypad or a keyboard, a touch screen, camera, optical scanner, network interface, web-based access, modem, a port, other known I/O devices or a combination of such I/O devices/interfaces 550. The touch screen may be activated with a stylus or a finger.

The I/O devices/interfaces 550 may include one or more devices for presenting output to a user, including, but not limited to, a graphics engine, a display (e.g., a display screen or monitor), one or more output drivers (e.g., display drivers), one or more audio speakers, and one or more audio drivers. In certain examples, I/O devices/interfaces 550 are configured to provide graphical data to a display for presentation to a user. The graphical data may be representative of one or more graphical user interfaces and/or any other graphical content as may serve a particular implementation.

The computing device 510 can further include a communication interface 560. The communication interface 560 can include hardware, software, or both. The communication interface 560 can provide one or more interfaces for communication (such as, for example, packet-based communication) between the computing device 510 and one or more additional computing devices 512 or one or more networks. For example, communication interface 560 may include a network interface controller (NIC) or network adapter for communicating with an Ethernet or other wire-based network or a wireless NIC (WNIC) or wireless adapter for communicating with a wireless network, such as a WI-FI.

Any suitable network and any suitable communication interface 560 may be used. For example, computing device 510 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, one or more portions of controller 500 or computing device 510 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination thereof. Computing device 510 may include any suitable communication interface 560 for any of these networks, where appropriate.

The computing device 510 may include a bus 570. The bus 570 can include hardware, software, or both that couples components of computing device 510 to each other. For example, bus 570 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination thereof.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiment disclosed herein are for purposes of illustration and are not intended to be limiting.

## Claims

1. A ureteroscope system, comprising:
a working channel (152); a handpiece (110) having a distal end region, a proximal end region, an interior region, a working channel port (118), a first portion of the working channel in fluid communication with the working channel port, and a cable port (120);
a catheter (104) extending from the distal end region of the handpiece, the catheter including a second portion of the working channel (152) in fluid communication with the working channel port and the first portion of the working channel, a distal end defining a working channel opening (107) in fluid communication with the working channel;
one or more sensors (209) positioned and configured to detect a position of at least a portion of a laser fiber; and
a processor in communication with the one or more sensors and configured to determine if an emitting end of the laser fiber configured to emit laser light is outside the working channel and spaced at least a predetermined distance from the distal end of the catheter such that laser light emitting from the emitting of the laser fiber does not damage the catheter and/or the handpiece,
wherein the one or more sensors include at least an image sensor positioned at the distal end of the catheter that is configured to detect at least a portion of the laser fiber has exited the working channel through the working channel opening in the distal end of the catheter with the processor being configured to determine if the emitting end of the laser fiber is outside the working channel and spaced at least the predetermined distance from the distal end by determining an amount of the laser fiber visible on a display (190) configured to display in real time the images collected from the image sensor.

2. The ureteroscope system of claim 1, wherein the handpiece includes a cable port and the ureteroscope system includes a cable connected or connectable to the handpiece at the cable port and configured to provide power to the one or more sensors and communicate with one or more electronic devices including the processor.

3. The ureteroscope system of claim 2, wherein the one or more electronic devices include at least the display configured to display in real time the images collected from the image sensor.

4. The ureteroscope system of claim 3, wherein the processor is configured to determine if the emitting end of the laser fiber is outside the working channel and spaced at least the predetermined distance from the distal end by determining a number or ratio of pixels on the display displaying a color of the laser fiber.

5. The ureteroscope system of claim **4,** wherein the processor is configured to coordinate an alert on the display if the emitting end of the laser fiber is outside the working channel and spaced at least the predetermined distance from the distal end of the catheter or if the emitting end of the laser fiber is not outside the working channel and/or not spaced at least the predetermined distance from the distal end of the catheter.

6. The ureteroscope system of any of claims 1 or 2, wherein the one or more sensors include at least one sensor positioned to detect the laser fiber or a jacket on the laser fiber entering the working channel and/or passing through the working channel.

7. The ureteroscope system of claim 6, wherein:
at least some of the first portion of the working channel passing through the interior region of the handpiece and is translucent or transparent; and
the at least one sensor is positioned in the interior region of the handpiece at least proximate to the first portion of the working channel that is translucent or transparent, the at least one sensor being configured to detect the laser fiber or the jacket on the laser fiber passing through the first portion of the working channel that is translucent or transparent.

8. The ureteroscope system of claim 7, wherein the processor is configured to determine if the emitting end of the laser fiber is outside the working channel and spaced at least the predetermined distance from the distal end of the catheter based on the at least one sensor detecting the emitting end and a predetermined length of the laser fiber or the jacket on the laser fiber passing through the first portion of the working channel that is translucent or transparent.

9. The ureteroscope system of claim 7, further comprising the laser fiber having a plurality of markers on the laser fiber or the jacket on the laser fiber, wherein the at least one sensor is configured to detect the plurality of markers and the processor is configured to determine if the emitting end of the laser fiber is outside the working channel and spaced at least the predetermined distance from the distal end of the catheter based on at least a predetermined number of markers of the plurality of markers passing the at least one sensor.

10. The ureteroscope system of claim 6, wherein the at least one sensor is positioned on the handpiece proximate to the working channel port, the at least one sensor being configured to detect the laser fiber or the jacket on the laser fiber entering the working channel through the working channel port.

11. The ureteroscope system of claim 10, wherein the processor is configured to determine if the emitting end of the laser fiber is outside the working channel and spaced at least the predetermined distance from the distal end of the catheter based on the at least one sensor detecting the emitting end and a predetermined length of the laser fiber or the jacket on the laser fiber entering the working channel through the working channel port.

12. The ureteroscope system of claim 10, further comprising the laser fiber having a plurality of markers on the laser fiber or the jacket on the laser fiber, wherein the at least one sensor is configured to detect the plurality of markers and the processor is configured to determine if the emitting end of the laser fiber is outside the working channel and spaced at least the predetermined distance from the distal end of the catheter based on at least a predetermined number of markers of the plurality of markers entering the working channel through the working channel port.

13. The ureteroscope system of any of claims 1-12, wherein the predetermined distance is about 3 mm to about 8 mm.

## Patentansprüche

1. Ein Ureteroskop-System, umfassend:
einen Arbeitskanal (152); ein Handstück (110) mit einem distalen Endbereich, einem proximalen Endbereich, einem Innenbereich, einem Arbeitskanalanschluss (118), einem ersten Abschnitt des Arbeitskanals in Fluidverbindung mit dem Arbeitskanalanschluss und ein Kabelanschluss (120); einen Katheter (104), der sich vom distalen Endbereich des Handstücks erstreckt, wobei der Katheter einen zweiten Abschnitt des Arbeitskanals (152) in Fluidverbindung mit dem Arbeitskanalanschluss und dem ersten Abschnitt des Arbeitskanals umfasst, wobei ein distales Ende eine Arbeitskanalöffnung (107) in Fluidverbindung mit dem Arbeitskanal vorgibt;
einem oder mehreren Sensoren (209), die so positioniert und konfiguriert sind, dass sie eine Position mindestens eines Abschnitts einer Laserfaser erfassen; und
ein Prozessor, der mit dem einen oder den mehreren Sensoren in Verbindung steht und so konfiguriert ist, dass er erkennt, ob sich ein Emissionsende der Laserfaser, die so konfiguriert ist, dass sie Laserlicht emittiert, außerhalb des Arbeitskanals befindet und mindestens einen vorbestimmten Abstand vom distalen Ende des Katheters aufweist, so dass das Laserlicht, das von der Laserfaser emittiert wird, den Katheter und/oder das Handstück nicht beschädigt,
wobei der eine oder die mehreren Sensoren mindestens einen Bildsensor umfassen, der am distalen Ende des Katheters positioniert und so konfiguriert ist, dass er mindestens einen Teil der Laserfaser erfasst, welcher den Arbeitskanal durch die Arbeitskanalöffnung im distalen Ende des Katheters verlassen hat, wobei der Prozessor so konfiguriert ist, dass er erkennt, ob sich das Emissionsende der Laserfaser außerhalb des Arbeitskanals und mindestens in einem vorbestimmten Abstand vom distalen Ende befindet, indem er einen Anteil der Laserfaser erkennt, der auf einer Anzeige (190) sichtbar ist, das so konfiguriert ist, dass es in Echtzeit die Bilder anzeigt, die vom Bildsensor erfasst wurden.

2. Das Ureteroskop-System nach Anspruch 1, wobei das Handstück einen Kabelanschluss umfasst und das Ureteroskop-System ein Kabel umfasst, das mit dem Handstück am Kabelanschluss verbunden oder verbindbar ist und so konfiguriert ist, dass es den einen oder die mehreren Sensoren mit Strom versorgt und mit einem oder mehreren elektronischen Geräten, einschließlich des Prozessors, verbunden ist.

3. Das Ureteroskop-System nach Anspruch 2, wobei die eine oder mehreren elektronischen Geräte zumindest die Anzeige umfasst, die so konfiguriert ist, dass sie die vom Bildsensor erfassten Bilder in Echtzeit anzeigt.

4. Das Ureteroskop-System nach Anspruch 3, wobei der Prozessor so konfiguriert ist, dass er erkennt, ob das Emissionsende der Laserfaser sich außerhalb des Arbeitskanals befindet und mindestens den vorbestimmten Abstand vom distalen Ende aufweist, indem er eine Anzahl oder ein Verhältnis von Pixeln auf der Anzeige bestimmt, die eine Farbe der Laserfaser anzeigen.

5. Das Ureteroskop-System nach Anspruch 4, wobei der Prozessor so konfiguriert ist, dass er eine Warnung auf der Anzeige koordiniert, wenn sich das Emissionsende der Laserfaser außerhalb des Arbeitskanals befindet und mindestens den vorbestimmten Abstand vom distalen Ende des Katheters aufweist, oder wenn sich das Emissionsende der Laserfaser nicht außerhalb des Arbeitskanals liegt und/oder nicht mindestens den vorbestimmten Abstand vom distalen Ende des Katheters aufweist.

6. Das Ureteroskop-System nach einem der Ansprüche 1 oder 2, wobei der eine oder die mehreren Sensoren mindestens einen Sensor umfassen, der so positioniert ist, dass er die Laserfaser oder eine Ummantelung der Laserfaser erfasst, die in den Arbeitskanal eintritt und/oder den Arbeitskanal durchläuft.

7. Das Ureteroskop-System nach Anspruch 6, wobei:
zumindest ein Teil des ersten Abschnitts des Arbeitskanals durch den Innenbereich des Handstücks verläuft und durchscheinend oder transparent ist; und
der mindestens eine Sensor im Innenbereich des Handstücks zumindest in der Nähe des ersten Abschnitts des Arbeitskanals positioniert ist, der lichtdurchlässig oder transparent ist, wobei der mindestens eine Sensor so konfiguriert ist, dass er die Laserfaser oder die Ummantelung der Laserfaser erkennt, die durch den ersten Abschnitt des Arbeitskanals verläuft, der lichtdurchlässig oder transparent ist.

8. Das Ureteroskop-System nach Anspruch 7, wobei der Prozessor so konfiguriert ist, dass er erkennt, ob sich auf der Grundlage des mindestens einen Sensors das Emissionsende der Laserfaser außerhalb des Arbeitskanals und in einem Abstand von mindestens dem vorbestimmten Abstand vom distalen Ende des Katheters befindet, und sich eine vorbestimmte Länge der Laserfaser oder der Ummantelung der Laserfaser durch den ersten Abschnitt des Arbeitskanals erstreckt, der lichtdurchlässig oder transparent ist.

9. Das Ureteroskop-System nach Anspruch 7, wobei weiter die Laserfaser eine Mehrzahl von Markern auf der Laserfaser oder der Ummantelung der Laserfaser aufweist, wobei der mindestens eine Sensor so konfiguriert ist, dass er die Mehrzahl von Markern erfasst, und der Prozessor so konfiguriert ist, dass er erkennt, ob sich das Emissionsende der Laserfaser außerhalb des Arbeitskanals befindet und mindestens den vorbestimmten Abstand vom distalen Ende des Katheters aufweist, basierend auf mindestens einer vorbestimmten Anzahl von Markern der mehreren Marker, die den mindestens einen Sensor passieren.

10. Das Ureteroskop-System nach Anspruch 6, wobei der mindestens eine Sensor auf dem Handstück in der Nähe der Arbeitskanalöffnung positioniert ist, wobei der mindestens eine Sensor so konfiguriert ist, dass er die Laserfaser oder die Ummantelung der Laserfaser erfasst, die durch die Arbeitskanalöffnung in den Arbeitskanal eintritt.

11. Das Ureteroskop-System nach Anspruch 10, wobei der Prozessor so konfiguriert ist, dass er erkennt, ob sich auf der Grundlage des mindestens einen Sensors das Emissionsende der Laserfaser außerhalb des Arbeitskanals und in einem Abstand von mindestens dem vorbestimmten Abstand vom distalen Ende des Katheters befindet, und eine vorbestimmte Länge der Laserfaser oder der Ummantelung der Laserfaser, die durch die Arbeitskanalöffnung in den Arbeitskanal eintritt.

12. Das Ureteroskop-System nach Anspruch 10, wobei ferner die Laserfaser eine Mehrzahl von Markern auf der Laserfaser oder der Ummantelung der Laserfaser umfasst, wobei der mindestens eine Sensor so konfiguriert ist, dass er die Mehrzahl von Markern erfasst, und der Prozessor so konfiguriert ist, dass er erkennt, ob das Emissionsende der Laserfaser sich außerhalb des Arbeitskanals befindet und mindestens den vorbestimmten Abstand vom distalen Ende des Katheters aufweist, basierend auf mindestens einer vorbestimmten Anzahl von Markern der Mehrzahl von Markern, die durch die Arbeitskanalöffnung in den Arbeitskanal eintreten.

13. Das Ureteroskop-System nach einem der Ansprüche 1 bis 12, wobei der vorbestimmte Abstand etwa 3 mm bis etwa 8 mm beträgt.

## Revendications

1. Système d'urétéroscope, comprenant :
un canal opérateur (152) ;
une pièce à main (110) comportant une région d'extrémité distale, une région d'extrémité proximale, une région intérieure, un orifice du canal opérateur (118), une première portion du canal opérateur en communication fluidique avec l'orifice du canal opérateur, et un orifice de câble (120) ;
un cathéter (104) s'étendant depuis la région d'extrémité distale de la pièce à main, le cathéter comprenant une deuxième portion du canal opérateur (152) en communication fluidique avec l'orifice du canal opérateur et la première portion du canal opérateur, une extrémité distale définissant une ouverture du canal opérateur (107) en communication fluidique avec le canal opérateur ;
un ou plusieurs capteurs (209) positionnés et configurés pour détecter une position d'au moins une portion d'une fibre laser ; et
un processeur en communication avec le ou les capteurs et configuré pour déterminer si une extrémité émettrice de la fibre laser, configurée pour émettre un rayonnement laser, est située à l'extérieur du canal opérateur et espacée d'au moins une distance prédéterminée par rapport à l'extrémité distale du cathéter de sorte que le rayonnement laser émis par l'extrémité émettrice de la fibre laser n'endommage pas le cathéter et/ou la pièce à main,
dans lequel le ou les capteurs comprennent au moins un capteur d'image positionné à l'extrémité distale du cathéter qui est configuré pour détecter qu'au moins une portion de la fibre laser est sortie du canal opérateur à travers l'ouverture du canal opérateur dans l'extrémité distale du cathéter, le processeur étant configuré pour déterminer si l'extrémité émettrice de la fibre laser est située à l'extérieur du canal opérateur et espacée d'au moins la distance prédéterminée par rapport à l'extrémité distale en déterminant une portion de la fibre laser visible sur un écran (190) configuré pour afficher en temps réel les images acquises à partir du capteur d'image.

2. Système d'urétéroscope selon la revendication 1, dans lequel la pièce à main comprend un orifice de câble et le système d'urétéroscope comprend un câble connecté ou connectable à la pièce à main au niveau de l'orifice de câble et configuré pour alimenter le ou les capteurs et communiquer avec un ou plusieurs dispositifs électroniques comprenant le processeur.

3. Système d'urétéroscope selon la revendication 2, dans lequel le ou les dispositifs électroniques comprennent au moins l'écran configuré pour afficher en temps réel les images acquises à partir du capteur d'image.

4. Système d'urétéroscope selon la revendication 3, dans lequel le processeur est configuré pour déterminer si l'extrémité émettrice de la fibre laser est située à l'extérieur du canal opérateur et espacée d'au moins la distance prédéterminée par rapport à l'extrémité distale en déterminant un nombre ou un rapport de pixels sur l'écran affichant une couleur de la fibre laser.

5. Système d'urétéroscope selon la revendication 4, dans lequel le processeur est configuré pour déclencher une alerte sur l'écran si l'extrémité émettrice de la fibre laser est située à l'extérieur du canal opérateur et espacée d'au moins la distance prédéterminée par rapport à l'extrémité distale du cathéter ou si l'extrémité émettrice de la fibre laser n'est pas située à l'extérieur du canal opérateur et/ou n'est pas espacée d'au moins la distance prédéterminée par rapport à l'extrémité distale du cathéter.

6. Système d'urétéroscope selon l'une quelconque des revendications 1 ou 2, dans lequel le ou les capteurs comprennent au moins un capteur positionné pour détecter la fibre laser ou une gaine sur la fibre laser entrant dans le canal opérateur et/ou passant à travers le canal opérateur.

7. Système d'urétéroscope selon la revendication 6, dans lequel :
au moins une partie de la première portion du canal opérateur passant à travers la région intérieure de la pièce à main est translucide ou transparente ; et
l'au moins un capteur est positionné dans la région intérieure de la pièce à main au moins à proximité de la première portion du canal opérateur qui est translucide ou transparente, l'au moins un capteur étant configuré pour détecter la fibre laser ou la gaine sur la fibre laser passant à travers la première portion du canal opérateur qui est translucide ou transparente.

8. Système d'urétéroscope selon la revendication 7, dans lequel le processeur est configuré pour déterminer si l'extrémité émettrice de la fibre laser est située à l'extérieur du canal opérateur et espacée d'au moins la distance prédéterminée par rapport à l'extrémité distale du cathéter sur la base de la détection, par l'au moins un capteur, de l'extrémité émettrice et d'une longueur prédéterminée de la fibre laser ou de la gaine sur la fibre laser passant à travers la première portion du canal opérateur qui est translucide ou transparente.

9. Système d'urétéroscope selon la revendication 7, comprenant en outre la fibre laser ayant une pluralité de repères sur la fibre laser ou la gaine sur la fibre laser, dans lequel l'au moins un capteur est configuré pour détecter la pluralité de repères et le processeur est configuré pour déterminer si l'extrémité émettrice de la fibre laser est située à l'extérieur du canal opérateur et espacée d'au moins la distance prédéterminée par rapport à l'extrémité distale du cathéter sur la base du passage d'au moins un nombre prédéterminé de repères, parmi la pluralité de repères, devant l'au moins un capteur.

10. Système d'urétéroscope selon la revendication 6, dans lequel l'au moins un capteur est positionné sur la pièce à main à proximité de l'orifice du canal opérateur, l'au moins un capteur étant configuré pour détecter la fibre laser ou la gaine sur la fibre laser entrant dans le canal opérateur à travers l'orifice du canal opérateur.

11. Système d'urétéroscope selon la revendication 10, dans lequel le processeur est configuré pour déterminer si l'extrémité émettrice de la fibre laser est située à l'extérieur du canal opérateur et espacée d'au moins la distance prédéterminée par rapport à l'extrémité distale du cathéter sur la base de la détection, par l'au moins un capteur, de l'extrémité émettrice et d'une longueur prédéterminée de la fibre laser ou de la gaine sur la fibre laser entrant dans le canal opérateur à travers l'orifice du canal opérateur.

12. Système d'urétéroscope selon la revendication 10, comprenant en outre la fibre laser ayant une pluralité de repères sur la fibre laser ou la gaine sur la fibre laser, dans lequel l'au moins un capteur est configuré pour détecter la pluralité de repères et le processeur est configuré pour déterminer si l'extrémité émettrice de la fibre laser est située à l'extérieur du canal opérateur et espacée d'au moins la distance prédéterminée par rapport à l'extrémité distale du cathéter sur la base de l'entrée d'au moins un nombre prédéterminé de repères, parmi la pluralité de repères, dans le canal opérateur à travers l'orifice du canal opérateur.

13. Système d'urétéroscope selon l'une quelconque des revendications 1 à 12, dans lequel la distance prédéterminée est d'environ 3 mm à environ 8 mm.
